# EUROPEAN PATENT APPLICATION

(11) **EP 4 464 290 A1**
(43) Date of publication of application: **20.11.2024**
(21) Application number: 22920428.4
(22) Date of filing: 01.11.2022
(51) Int. Cl.: A61F 9/007, A61F 2/14

(54) **RESIN CHIP FOR TISSUE IMPLANTATION, TISSUE IMPLANTATION DEVICE, RETINAL TISSUE IMPLANTATION METHOD AND RETINAL TISSUE IMPLANTATION KIT**

(30) Priority: 12.01.2022 JP 2022003381
(71) Applicant: RIKEN, Wako-shi, Saitama 351-0198 (JP); Sumitomo Pharma Co., Ltd., Osaka-shi, Osaka 541-0045 (JP)
(72) Inventor: MANDAI, Michiko, Wako-shi, Saitama 351-0198 (JP); KURIMOTO, Yasuo, Wako-shi, Saitama 351-0198 (JP); KUWAHARA, Atsushi, Kobe-shi, Hyogo 650-0047 (JP); NAKAGAWA, Takashi, Suita-shi, Osaka 564-0053 (JP); MANABE, Koichiro, Tokyo 103-6012 (JP)
(74) Representative: Zimmermann & Partner Patentanwälte mbB
(86) International application number: PCT/JP2022/040856
(87) International publication number: WO 2023/135906

(57) **Abstract**

A tissue transplantation resin tip and the like that allow easy transplantation of a tissue sucked inside are provided. A tissue transplantation resin tip 10 according to the present invention includes a needle tube 1 that can be inserted into an eyeball. The needle tube includes a main body portion 11 that extends linearly, and a front end portion 12 that is bent with respect to the main body portion and extends linearly, and that discharges a tissue T from a front end 121. A length L1 of the needle tube is 25 mm or more and 50 mm or less, a cross-sectional external shape of the front end portion has an oval shape having a major axis D1 of 0.8 mm or more and 1.5 mm or less, and a minor axis D2 of 0.5 mm or more and 1.0 mm or less, the front end of the front end portion is a bevel surface that extends in a direction substantially perpendicular to the main body portion, and that has a bevel angle Θ1 of 50° or more and 85° or less, the hardness of the front end portion is 0.15 N or more and 0.30 N or less, and the hardness of the center of the needle tube is 0.80 N or more and 1.50 N or less.

## Description

### [Technical Field]

The present invention relates to a tissue transplantation resin tip used for transplanting a tissue under a retina in an eyeball, a tissue transplantation device including the same, a retina tissue transplantation method using the same, and a retina tissue transplantation kit. Particularly, the present invention relates to a tissue transplantation resin tip, a tissue transplantation device, a retina tissue transplantation method, and a retina tissue transplantation kit that allow easy transplantation of a tissue sucked inside.

### [Background Art]

Conventionally, tissue transplantation resin tips have been proposed that are used for transplanting a tissue, such as a retina tissue, under a retina in an eyeball (for example, refer to Patent Literature 1 and Supplementary Appendix of Non Patent literature 1).

These tissue transplantation resin tips are used as follows. That is, after a tissue is sucked inside a tissue transplantation resin tip, the tissue transplantation resin tip is inserted into a space (subretinal space) under a retina in an eyeball of a patient, and the tissue is discharged to the space under the retina from a front end of the tissue transplantation resin tip, thereby transplanting the tissue.

However, conventional tissue transplantation resin tips did not necessarily provide sufficient ease for tissue transplantation, and a tissue transplantation resin tip has been desired that provides more ease for transplantation.

### [Citation List]

### [Patent Literature]

[Patent Literature 1]
US5941250A

### [Non Patent Literature]

[Non Patent Literature 1]
M. Mandai, et al., "Autologous Induced Stem-Cell-Derived Retinal Cells for Macular Degeneration", The New England Journal of Medicine, March 16, 2017, 376, p. 1038-1046

### [Summary of Invention]

### [Technical Problem]

An object of the present invention is to provide a tissue transplantation resin tip, a tissue transplantation device, a retina tissue transplantation method, and a retina tissue transplantation kit that allow easy transplantation of a tissue sucked inside.

### [Solution to Problem]

In order to achieve the object, the inventors have conducted extensive studies by repeating prototyping of a tissue transplantation resin tip. As a result, the inventors have found that it is possible to obtain a tissue transplantation resin tip that allows easy transplantation of a tissue sucked inside under a retina of an eyeball, by optimizing the shape, dimension, and hardness of a needle tube provided to the tissue transplantation resin tip, and have completed the present invention.

That is, in order to achieve the object, the present invention provides a tissue transplantation resin tip to be used for transplanting a tissue under a retina in an eyeball, the tissue transplantation resin tip comprising: a needle tube to be inserted into an eyeball, wherein the needle tube includes a main body portion that extends linearly, and a front end portion that is bent with respect to the main body portion and extends linearly, and that discharges the tissue from a front end, a length of the needle tube is 25 mm or more and 50 mm or less, a cross-sectional external shape of the front end portion is an oval shape having a major axis of 0.8 mm or more and 1.5 mm or less, and a minor axis of 0.5 mm or more and 1.0 mm or less, the front end of the front end portion is a bevel surface that extends in a direction substantially perpendicular to the main body portion, and that has a bevel angle of 50° or more and 85° or less, and a hardness of the front end portion is 0.15 N or more and 0.30 N or less, and a hardness of a center of the needle tube is 0.80 N or more and 1.50 N or less.

In the present invention, a "tissue" means a cell population structure body that has a structure in which one or more types of cells with different forms and properties are three-dimensionally arranged in a certain pattern.

Additionally, in the present invention, "the length of a needle tube" means the dimension in a direction parallel to the longitudinal direction (extending direction) of a main body portion (the dimension in a direction parallel to a central axis of the main body portion of a needle tube) between a front end of the needle tube (the front end of a front end portion of the needle tube), and a rear end of the needle tube (the rear end of the main body portion of the needle tube). The "front end" means an end of the needle tube that is first inserted into an eyeball, and the "rear end" means the opposite end. In one aspect of the present invention, when a tissue transplantation resin tip includes a "connecting portion", which will be described later, the "rear end" of the needle tube means a front end of the connecting portion, or the boundary between the needle tube and the connecting portion.

Additionally, in the present invention, "the cross-sectional external shape of the front end portion" means the cross-sectional external shape in a direction perpendicular to the longitudinal direction (extending direction) of the front end portion of the needle tube.

Additionally, in the present invention, "the direction substantially perpendicular to the main body portion" means a direction substantially perpendicular to the central axis of the main body portion of the needle tube. In one aspect of the present invention, "the direction substantially perpendicular to the main body portion" means a direction that is 80° or more (preferably, 85° or more, 87° or more, 88° or more, or 89° or more), and 100° or less (preferably, 95° or less, 93° or less, 92° or less, or 91° or less) with respect to the central axis of the main body portion of the needle tube.

Additionally, in the present invention, a "bevel angle" means an acute angle formed by the front end (bevel surface) of the front end portion of the needle tube and an external surface of the front end portion of the needle tube that is adjacent to the front end.

Further, in the present invention, a "hardness" means a load required to displace a portion to be measured for hardness by 5 mm in a state where the rear end of the tissue transplantation resin tip is fixed.

According to a tissue transplantation resin tip according to the present invention, a front end portion of a needle tube is bent with respect to a main body portion of the needle tube and extends linearly, and a front end of the front end portion of the needle tube is a bevel surface that extends in a direction substantially perpendicular to the main body portion of the needle tube, and that has a bevel angle of 50° or more and 85° or less (for example, approximately 60°), preferably 60° or more and 85° or less (for example, approximately 75° (75° ± 3°, 75° ± 2°, 75° ± 1°)). Therefore, it becomes easier to insert the front end portion of the needle tube into a space under a retina in an eyeball of a patient and to lift up the retina, and it also becomes easier to discharge a tissue from the front end of the front end portion of the needle tube.

Additionally, according to the tissue transplantation resin tip according to the present invention, the hardnesses of the front end portion of the needle tube is 0.15 N or more and 0.30 N or less, and the hardnesses in the center (the center in the longitudinal direction) of the needle tube is 0.80 N or more and 1.50 N or less. Therefore, shaking of the needle tube within the eyeball can be prevented. Note that a person skilled in the art can manufacture a tissue transplantation resin tip that includes the center and the front end portion of the needle tube whose hardnesses are different from each other. As one aspect, it is possible to manufacture a tissue transplantation resin tip having different hardness depending on a portion as described above by designing the thickness of resin and the cross-sectional external shape to become smaller toward the front end portion.

In the tissue transplantation resin tip according to the present invention, in addition to having the above-described characteristics of the bevel angle and the hardness, the length of the needle tube and the shape of the cross-sectional external shape of the front end portion of the needle tube are defined in the aforementioned predetermined ranges. Accordingly, it is possible to easily and reliably transplant a tissue sucked inside the needle tube under a retina of interest.

Preferably, the hardness of the front end portion is 0.20 N or more and 0.30 N or less.

According to the above-described preferable configuration, shaking of the needle tube within an eyeball can be further prevented.

Preferably, a length of the front end portion is 2 mm or more and less than 6 mm.

In the above-described preferable configuration, "the length of the front end portion" means the dimension in a direction parallel to the longitudinal direction (extending direction) of the front end portion (the dimension in a direction parallel to the central axis of the front end portion of the needle tube) between the front end of the front end portion of the needle tube, and the rear end of the front end portion of the needle tube.

According to the above-described preferable configuration, it is possible to more easily and reliably transplant a tissue sucked inside the needle tube under a retina of interest.

Preferably, the front end portion is bent so as to form an angle of 120° or more and 160° or less with respect to the main body portion. More preferably, the front end portion is bent so as to form an angle of 120° or more (even more preferably 130° or more) and 150° or less with respect to the main body portion.

According to the above-described preferable configuration, it becomes easier to insert the front end portion of the needle tube into a space under a retina in an eyeball of a patient along the retina.

Preferably, an outer diameter of the center of the needle tube is 1.5 mm or more and 3.5 mm or less.

According to the above-described preferable configuration, shaking of the needle tube within an eyeball can be further prevented.

Preferably, the needle tube has transparency that allows the tissue moving inside the needle tube to be visually recognized from an outside.

According to the above-described preferable configuration, since the position of the tissue in the needle tube can be visually recognized from the outside, the tissue can be more easily transplanted.

The tissue transplantation resin tip according to the present invention is formed from at least one type of resin of, for example, polyolefin resin (polypropylene, polyethylene, polymethylpentene), cycloolefin resin [cycloolefin polymer (COP), cycloolefin copolymer (COC)], polyester resin (polyethylene terephthalate), fluorine resin [polytetrafluoroethylene (PTFE), perfluoroalkoxyalkane (PFA), perfluoroethylene-propene copolymer (FEP), ethylene tetrafluoroethylene copolymer (ETFE), polyvinylidene fluoride (PVDF), polychlorotrifluoroethylene (PCTFE), or ethylene chlorotrifluoroethylene copolymer (ECTFE)], polycarbonate, and polyimide.

Preferably, the tissue transplantation resin tip according to the present invention comprises a stopper that prevents movement of the tissue toward a rear end of the needle tube in the main body portion. A person skilled in the art can freely design the position at which the stopper is provided in the main body portion (the position at which the internal space of the main body portion is narrowed), the shape of the stopper, and the length of the stopper. As one aspect, for example, the stopper can be provided at a position that is 10 mm to 30 mm from the front end of the needle tube. As the stopper, for example, a tubular member made of resin or stainless steel that is inserted into the main body portion of the needle tube or that is integrally molded with the main body portion can be used. Additionally, it is also possible to provide, for example, a reduced diameter portion in which the surface of the main body portion of the needle tube is pressed to narrow the internal space, and to make this reduced diameter portion function as a stopper.

According to the above-described preferable configuration, since the tissue does not move toward the rear end beyond the position at which the stopper is provided, the tissue is not excessively sucked inside the needle tube, and the tissue can be more reliably transplanted.

Preferably, the tissue is a retina tissue.

In the above-described preferable configuration, a "retina tissue" means a tissue in which one type or a plurality of types of retina cells, such as photoreceptors, horizontal cells, bipolar cells, amacrine cells, retinal ganglion cells, retinal pigment epithelial cells, their precursor cells, or retinal precursor cells, constituting each retinal layer in the biological retina are three-dimensional arranged in layers. As one aspect, one or more cells of the above-described retina cells may form a single layer or a plurality of layers in a certain pattern. The layers included in the retina tissue may also be called a retinal pigment epithelium, an outer limiting membrane, a photoreceptor layer (outer nuclear layer), an outer plexiform layer, an inner nuclear layer, an inner plexiform layer, a ganglion cell layer, a nerve fiber layer, and an inner limiting membrane.

The retina tissue may be a retina tissue excised from a living body (for example, a fetus), or may be the retina tissue differentiated from autologous or allogeneic pluripotent stem cells (for example, embryonic stem cells (ES cells), an induced pluripotent stem cell (iPS cells)). The retina tissue may be the retina tissue including a neural retina (including photoreceptors).

A method of preparing a retina tissue from a living body is well known for a person skilled in the art. Specifically, a retina tissue can be cut out under anesthesia.

A method for inducing differentiation of pluripotent stem cells into a retina tissue includes, but not particularly limited to, the methods disclosed in literatures such as WO2011/055855, WO2013/077425, WO2015/025967, WO2016/063985, WO2016/063986, WO2017/183732, "PLoS One. 2010 Jan 20;5(1):e8763.", "Stem Cells. 2011 Aug;29(8):1206-18.", "Proc Natl Acad Sci USA. 2014 Jun 10;111 (23):8518-23", "Nat Commun. 2014 Jun 10;5:4047", WO2012/173207, WO2015/053375, WO2015/053376, WO2015/068505, WO2017/043605, "Stem Cell Reports, 2 (2), 205-218 (2014)", and "Cell Stem Cell, 10 (6), 771-785" (2012)" can be listed.

The size of the tissue is not particularly limited as long as the tissue can move inside the needle tube. For example, it is preferable that the tissue having a minor axis of 400 µm or more and 800 µm or less, and a thickness of 400 µm or more and 600 µm or less can move inside the needle tube. A retina tissue of the above-described size can be prepared by cutting out the retina tissue that has been prepared with the aforementioned method.

The tissue transplantation resin tip according to the present invention can be connected to, for example, a syringe to suck and discharge a tissue, but is not limited to this.

As one aspect, an injection syringe is connected to the tissue transplantation resin tip according to the present invention, and a tissue is sucked inside the needle tube of the tissue transplantation resin tip by pulling the injection syringe. By pressing the injection syringe, the tissue is discharged from the inside of the needle tube of the tissue transplantation resin tip. As another aspect of the discharging method, a tissue may be discharged by using an injection kit connected to a cataract/vitreous surgery device. Specifically, the tissue transplantation resin tip that has sucked a tissue into the needle tube is removed from an injection syringe, and is connected to the injection kit. Then, after inserting the needle tube of the tissue transplantation resin tip into a space under a retina in an eyeball of a patient, the tissue is discharged to the space under the retina from inside the needle tube by operating the injection kit with the cataract/vitreous surgery device.

As another aspect, a syringe of the injection kit connected to the cataract/vitreous surgery device may be directly connected to the tissue transplantation resin tip according to the present invention. Since the cataract/vitreous surgery device is usually provided with a suction function, this suction function is actuated to suck a tissue into the needle tube of the tissue transplantation resin tip. Thereafter, the tissue is discharged to the space under the retina from inside the needle tube with the same method as mentioned above.

On the other hand, when the tissue transplantation resin tip that has sucked the tissue inside the needle tube is reconnected to the injection kit as mentioned above, there is a possibility that the tissue is exposed to the outside environment, and the position of the tissue within the tissue transplantation resin tip is shifted due to the reconnection. Accordingly, it is more preferable to connect the syringe via a stopcock, which will be described later. Additionally, even when the tissue transplantation resin tip is connected to the injection kit from the beginning, there is a limit to a suction control function, and it is difficult to precisely control the position of the tissue in the tissue transplantation resin tip. Therefore, it is preferable to use the stopcock, which will be described later.

Further, in order to achieve the object, the present invention provides a tissue transplantation device, comprising: a stopcock; the tissue transplantation resin tip configured to be connected to a port of the stopcock; and a syringe configured to be connected to another port of the stopcock. The tissue transplantation resin tip and the syringe may not be directly connected to each port of the stopcock, but may be connected to each port via a tube (for example, an "Ocular Irrigation Tube" REF# 169-30L-6 manufactured by Eagle Labs, LLC) or the like.

In the present invention, a three-way stopcock or a two-way stopcock can be used as the "stopcock."

According to the tissue transplantation device according to the present invention, for example, when a three-way stopcock is provided as the stopcock, the three-way stopcock can be used as follows. That is, the tissue transplantation resin tip is connected to one port of the three-way stopcock, and a syringe is connected to another port. Then, a tissue is sucked inside the needle tube of the tissue transplantation resin tip by turning on (opening) the port connected to the tissue transplantation resin tip and the port connected to the syringe to pull the syringe. Next, the remaining port of the three-way stopcock is connected to, for example, an injection kit, the injection kit is connected to a cataract/vitreous surgery device, and the port connected to the tissue transplantation resin tip and the port connected to the injection kit are turned on (opened). Next, after inserting the needle tube of the tissue transplantation resin tip into the space under the retina in the eyeball of the patient, a tissue is discharged to the space under the retina from inside the needle tube by operating the injection kit with the cataract/vitreous surgery device. With the above operation, the tissue can be transplanted. The tissue transplantation resin tip, the syringe, and the injection kit may be directly connected to the respective ports of the three-way stopcock, or may be connected to the respective ports of the three-way stopcock via a tube (for example, an "Ocular Irrigation Tube" REF# 169-30L-6 manufactured by Eagle Labs, Inc.).

Additionally, according to the tissue transplantation device according to the present invention, for example, when a two-way stopcock is provided as the stopcock, the two-way stopcock can be used as follows. That is, the tissue transplantation resin tip is connected to one port of the two-way stopcock, and a syringe is connected to another port. Then, a tissue is sucked inside the needle tube of the tissue transplantation resin tip by turning on (opening) the port connected to the tissue transplantation resin tip and the port connected to the syringe to pull the syringe. Next, after turning off (closing) the port connected to the syringe to remove the syringe, for example, this port is connected to an injection kit, and the injection kit is connected to a cataract/vitreous surgery device to turn on (open) this port. Next, after inserting the needle tube of the tissue transplantation resin tip into the space under the retina in the eyeball of the patient, a tissue is discharged to the space under the retina from inside the needle tube by operating the injection kit with the cataract/vitreous surgery device. With the above operation, the tissue can be transplanted. The tissue transplantation resin tip, the syringe, and the injection kit may be directly connected to the respective ports of the two-way stopcock, or may be connected to the respective ports of the two-way stopcock via a tube (for example, an "Ocular Irrigation Tube" REF# 169-30L-6 manufactured by Eagle Labs, Inc.).

Further, in order to achieve the object, the present invention provides a retina tissue transplantation method of transplanting a retina tissue under a retina in an eyeball of a patient who suffers from a disease based on a disorder of or a damage to the retina tissue, the retina tissue transplantation method comprising: a step of sucking the retina tissue inside the needle tube of the tissue transplantation resin tip by pulling the syringe using the tissue transplantation device; a step of inserting the needle tube that has sucked the retina tissue into a space under the retina in the eyeball of the patient; and a step of discharging the retina tissue to the space under the retina from inside the needle tube.

Diseases based on disorders of retina tissue include, for example, ophthalmic diseases such as retinal degenerative diseases, macular degeneration, age-related macular degeneration, retinitis pigmentosa, glaucoma, corneal diseases, retinal detachment, central serous retinal choroidopathy, cone dystrophy, cone rod dystrophy. Diseases based on damage to retina tissue (damaged conditions of retina tissue) include for example, states where photoreceptors, retinal pigment epithelial cells, or the like are degenerated, atrophied, or dead.

Further, in order to achieve the object, the present invention provides a retina tissue transplantation kit, comprising: a retina tissue; and the tissue transplantation resin tip.

The retina tissue transplantation kit according to the present invention may further include at least one or more of a syringe, an injection kit, and a tube (for example, an "Ocular Irrigation Tube" REF# 169-30L-6 manufactured by Eagle Labs, Inc.).

Summarizing the above, the present invention relates to the following matters.
[1] A tissue transplantation resin tip to be used for transplanting a tissue under a retina in an eyeball, the tissue transplantation resin tip comprising: a needle tube to be inserted into an eyeball, wherein the needle tube includes a main body portion that extends linearly, and a front end portion that is bent with respect to the main body portion and extends linearly, and that discharges the tissue from a front end, a length of the needle tube is 25 mm or more and 50 mm or less, a cross-sectional external shape of the front end portion is an oval shape having a major axis of 0.8 mm or more and 1.5 mm or less, and a minor axis of 0.5 mm or more and 1.0 mm or less, the front end of the front end portion is a bevel surface that extends in a direction substantially perpendicular to the main body portion, and that has a bevel angle of 50° or more and 85° or less, and a hardness of the front end portion is 0.15 N or more and 0.30 N or less, and a hardness of a center of the needle tube is 0.80 N or more and 1.50 N or less.
[2] The tissue transplantation resin tip according to [1], wherein the hardness of the front end portion is 0.20 N or more and 0.30 N or less.
[3] The tissue transplantation resin tip according to [1] or [2], wherein a length of the front end portion is 2 mm or more and less than 6 mm.
[4] The tissue transplantation resin tip according to any one of [1] to [3], wherein the front end portion is bent so as to form an angle of 120° or more and 160° or less with respect to the main body portion.
[5] The tissue transplantation resin according to any one of [1] to [4], wherein an outer diameter of the center of the needle tube is 1.5 mm or more and 3.5 mm or less.
[6] The tissue transplantation resin tip according to any one of [1] to [5], wherein the needle tube has transparency that allows the tissue moving inside the needle tube to be visually recognized from an outside.
[7] The tissue transplantation resin tip according to any one of [1] to [6], wherein the tissue transplantation resin tip is formed from at least one type of resin of polyolefin resin, cycloolefin resin, polyester resin, fluorine resin, polycarbonate, and polyimide.
[8] The tissue transplantation resin tip according to any one of [1] to [7], comprising: a stopper that prevents movement of the tissue toward a rear end of the needle tube in the main body portion.
[9] The tissue transplantation resin tip according to any one of [1] to [8], wherein the tissue is a retina tissue, and the retina tissue having a minor axis of 400 µm or more and 800 µm or less, and a thickness of 400 µm or more and 600 µm or less can move inside the needle tube.
[10] A tissue transplantation device, comprising: a stopcock; the tissue transplantation resin tip according to any one of [1] to [9] configured to be connected to a port of the stopcock; and a syringe configured to be connected to another port of the stopcock.
[11] A retina tissue transplantation method of transplanting a retina tissue under a retina in an eyeball of a patient who suffers from a disease based on a disorder of or a damage to the retina tissue, the retina tissue transplantation method comprising: a step of sucking the retina tissue inside the needle tube of the tissue transplantation resin tip by pulling the syringe using the tissue transplantation device according to [10]; a step of inserting the needle tube that has sucked the retina tissue into a space under the retina in the eyeball of the patient; and a step of discharging the retina tissue to the space under the retina from inside the needle tube.
[12] A retina tissue transplantation kit, comprising: a retina tissue; and the tissue transplantation resin tip according to any one of [1] to [8].

### [Advantageous Effects of Invention]

According to the present invention, it is possible to provide a tissue transplantation resin tip, a tissue transplantation device, a retina tissue transplantation method, and a retina tissue transplantation kit that allow easy transplantation of a tissue sucked inside.

### [Brief Description of Drawings]

[Figures 1A to 1D] Figures 1A to 1D are diagrams illustrating the schematic configuration of a tissue transplantation resin tip according to an embodiment of the present invention.
[Figure 2] Figure 2 is a diagram describing a method of measuring the hardness of a needle tube illustrated in Figures 1A to 1D.
[Figure 3] Figure 3 is a perspective view schematically illustrating an example of the schematic configuration of a tissue to be transplanted by the tip illustrated in Figures 1A to 1D.
[Figures 4A and 4B] Figures 4A and 4B are diagrams illustrating the schematic configuration of a modification of the tip illustrated in Figures 1A to 1D.
[Figure 5] Figure 5 is a plan view illustrating the schematic configuration of a tissue transplantation device according to an embodiment of the present invention.
[Figure 6] Figure 6 is a diagram illustrating an example of a transplantation method of a tissue using the device illustrated in Figure 5.
[Figures 7A and 7B] Figures 7A and 7B are diagrams describing the content of evaluation tests using each of tips.
[Figure 8] Figure 8 is a side view illustrating the schematic configuration of a tip described in Non Patent Literature 1.

### [Description of Embodiments]

Hereinafter, an embodiment of the present invention will be described with reference to the accompanying drawings as appropriate. Note that each diagram is a representation for reference, and the dimensions, scales, and shapes of the components represented in each figure may be different from the actual dimensions, scales, and shapes.

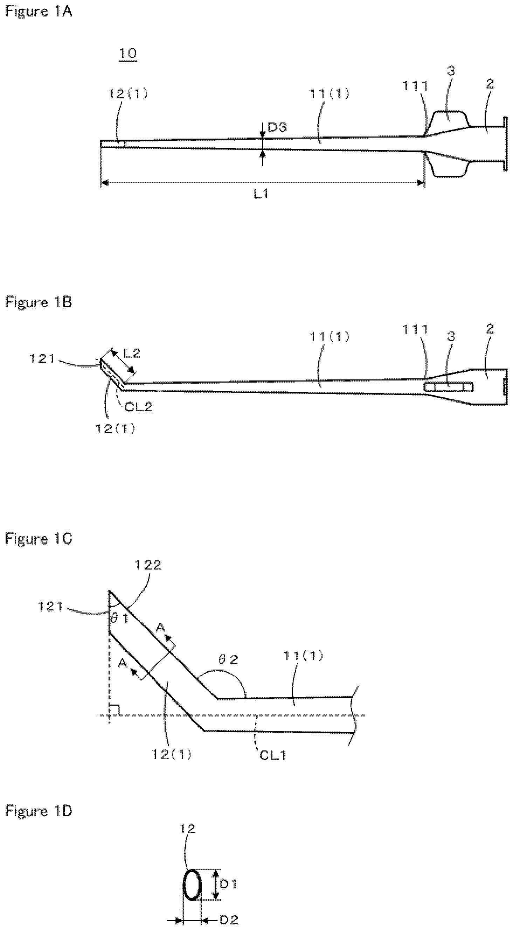

Figures 1A to 1D are diagrams illustrating the schematic configuration of a tissue transplantation resin tip (hereinafter abbreviated as "tip" as appropriate) according to an embodiment of the present invention. Figure 1A is a plan view illustrating the schematic configuration of the tip according to the present embodiment (a diagram seen from a direction along a flat surface that passes a main body portion and a front end portion of a needle tube included in the tip). Figure 1B is a side view illustrating the schematic configuration of the tip according to the present embodiment (a diagram seen from a direction perpendicular to the flat surface that passes the main body portion and the front end portion of the needle tube included in the tip). Figure 1C is a side view illustrating the schematic configuration of the vicinity of the front end portion of the tip according to the present embodiment in an enlarged manner. Figure 1D is a cross-sectional view taken along the arrows AA illustrated in Figure 1C.

A tip 10 according to the present embodiment illustrated in Figures 1A to 1D is a resin tip used for transplanting a tissue under a retina in an eyeball. Specifically, the tip 10 according to the present embodiment is preferably used for transplanting a retina tissue under a retina in an eyeball of a patient who suffers from a disease based on a disorder of or a damage to a retina tissue. As illustrated in Figures 1A to 1D, the tip 10 according to the present embodiment includes a needle tube 1 that may be inserted into an eyeball. Additionally, the tip 10 according to the present embodiment includes a hollow connecting portion 2 that is connected to a rear end of the needle tube 1 and that communicates with the needle tube 1, and a blade portion 3 provided to an external surface of the connecting portion 2. Note that a "front end" means an end of the needle tube 1 on the side that is first inserted into an eyeball (the left side in Figures 1A to 1C), and the "rear end" means an end on the opposite side (the right side in Figures 1A to 1C).

The blade portion 3 has a function of ensuring connection of the tip 10 to a port 20a of a stopcock 20, which will be described later. Additionally, the blade portion 3 can also be used as a mark of the rear end of the needle tube 1 at the time when the needle tube 1 is inserted into an eyeball.

The needle tube 1 includes a main body portion 11 that extends linearly, and a front end portion 12 that is bent with respect to the main body portion 11 and extends linearly, and that discharges a tissue from a front end 121. The main body portion 11 of the needle tube 1 of the present embodiment has a circular cross-sectional external shape, and has a tapered external shape in which the outer diameter of the cross-section becomes smaller toward the front end portion 12.

Since the main body portion 11 has the tapered external shape, the hardness of the rear end of the needle tube 1 is increased, and shaking of the needle tube 1 within an eyeball can be effectively prevented.

A length L1 of the needle tube 1 is 25 mm or more and 50 mm or less. Here, the length L1 of the needle tube 1 means a dimension in a direction parallel to the longitudinal direction (extending direction) of the main body portion 11 (a dimension in a direction parallel to a central axis CL1 of the main body portion 11 of the needle tube 1) between the front end 121 of the needle tube 1 (the front end 121 of the front end portion 12 of the needle tube 1), and a rear end 111 of the needle tube 1 (the rear end 111 of the main body portion 11 of the needle tube 1). Note that the rear end 111 of the needle tube 1 coincides with a front end of the blade portion 3.

The cross-sectional external shape of the front end portion 12 of the needle tube 1 (the cross-sectional external shape in a direction perpendicular to the longitudinal direction (extending direction) of the front end portion 12 of the needle tube 1) is an oval shape whose major axis is on the side surface side of the front end portion 12 (the side seen from the direction perpendicular to the flat surface that passes the main body portion 11 and the front end portion 12 of the needle tube 1), a major axis D1 is 0.8 mm or more and 1.5 mm or less, and a minor axis D2 is 0.5 mm or more and 1.0 mm or less.

The front end 121 of the front end portion 12 of the needle tube 1 is a bevel surface that extends in a direction substantially perpendicular to the main body portion 11, and has a bevel angle Θ1 of 50° or more and 85° or less. Here, as illustrated in Figure 1C, the "direction substantially perpendicular to the main body portion 11" means a direction substantially perpendicular to the central axis CL1 of the main body portion 11 of the needle tube 1. Additionally, the "bevel angle Θ1" means an acute angle formed by the front end 121 (the bevel surface) of the front end portion 12 of the needle tube 1 and an external surface 122 of the front end portion 12 of the needle tube 1 that is adjacent to the front end 121.

Since the front end portion 12 of the needle tube 1 is bent with respect to the main body portion 11 of the needle tube 1 and extends linearly, and the front end 121 of the front end portion 12 of the needle tube 1 is the bevel surface that extends in the direction substantially perpendicular to the main body portion 11 of the needle tube 1, and has the bevel angle Θ1 of 50° or more and 85° or less, it becomes easier to insert the front end portion 12 of the needle tube 1 into a space under a retina in an eyeball of a patient to raise the retina, and it also becomes easier to discharge a tissue from the front end 121 of the front end portion 12 of the needle tube 1.

The hardness of the front end portion 12 of the needle tube 1 is 0.15 N or more and 0.30 N or less (preferably, 0.20 N or more and 0.30 N or less), and the hardness of the center of the needle tube 1 (a portion that is the center of the needle tube 1 in the longitudinal direction, and that is located at a distance of 1/2 of the length L1 from the front end 121 of the needle tube 1 and has an outer diameter D3 in Figure 1A) is 0.80 N or more and 1.50 N or less. Here, the "hardness" means the load required to displace a portion (the front end portion 12 of the needle tube 1, the center of the needle tube 1) for measuring the hardness by 5 mm in a state where the rear end of the tip 10 is fixed.

By setting the hardness of the front end portion 12 and the hardness of the center of the needle tube 1 within the above-described ranges, shaking of the needle tube 1 within an eyeball can be prevented.

Note that the hardness of each portion of the needle tube 1 can be adjusted by appropriately setting the forming material, wall thickness, and the like of the needle tube 1.

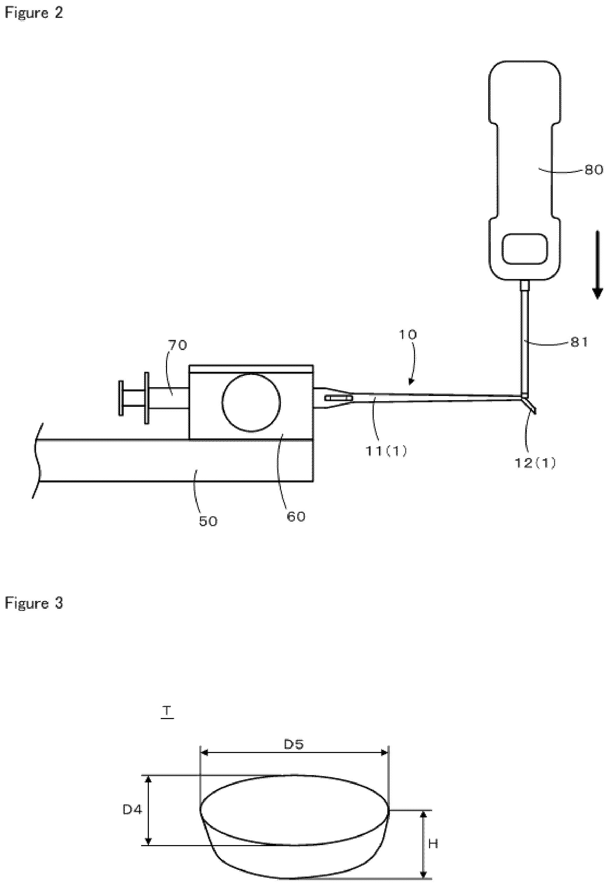

Figure 2 is a diagram for describing a method of measuring the hardness of the needle tube 1. Figure 2 illustrates a case where the hardness of the front end portion 12 of the needle tube 1 is measured. As illustrated in Figure 2, when measuring the hardness of the needle tube 1, a flat support table 50, a small size vise (mini vice) 60, a syringe (for example, a 0.5 mL syringe manufactured by Bethel Co., Ltd.) 70, a force gauge (for example, a digital force gauge "DVS-5N" manufactured by IMADA Co., Ltd.) 80, and a V-type attachment 81 attached to the force gauge 80 are used.

When measuring the hardness of the front end portion 12 of the needle tube 1, the small size vise 60 is fixed to the support table 50, and the syringe 70 is attached to the small size vise 60. Then, the tip 10 is attached to a lure portion (not illustrated) of the syringe 70 such that the front end portion 12 faces downward. In this state (a state where the rear end of the tip 10 is fixed), the V-type attachment 81 is caused to abut the front end portion 12 (specifically, the rear end of the front end portion 12) from above, the force gauge 80 and the V-type attachment 81 are displaced downward by 5 mm (accordingly, the front end portion 12 is also displaced downward by 5 mm), and the load (peak load) measured by the force gauge 80 in this state is used as the hardness of the front end portion 12 of the needle tube 1. When measuring the hardness of the center of the needle tube 1, the V-type attachment 81 is caused to abut the center of the needle tube 1 from above, the force gauge 80 and the V-type attachment 81 are displaced downward by 5 mm (accordingly, the center of the needle tube 1 is also displaced downward by 5 mm), and the load (peak load) measured by the force gauge 80 in this state is used as the hardness of the center of the needle tube 1.

In the tip 10 according to the present invention, in addition to having the above-described characteristics of the bevel angle Θ1 and the hardness, the length L1 of the needle tube 1 and the shape of the cross-sectional external shape of the front end portion 12 of the needle tube 1 are defined in the aforementioned predetermined ranges. Accordingly, it is possible to easily and reliably transplant a tissue sucked inside the needle tube 1 under a retina of interest.

A length L2 of the front end portion 12 of the needle tube 1 is 2 mm or more and less than 6 mm (less than 4 mm as one aspect). Here, the length L2 of the front end portion 12 of the needle tube 1 means a dimension in a direction parallel to the longitudinal direction (extending direction) of the front end portion 12 (a dimension in a direction parallel to a central axis CL2 of the front end portion 12 of the needle tube 1) between the front end 121 of the front end portion 12 of the needle tube 1 and the rear end of the front end portion 12 of the needle tube 1 (the front end of the main body portion 11).

The front end portion 12 of the needle tube 1 is bent so as to form an angle (bending angle) θ2 of 120° or more and 160° or less with respect to the main body portion 11 of the needle tube 1.

By setting the bending angle θ2 within the above-described range, it becomes easier to insert the front end portion 12 of the needle tube 1 into a space under a retina in an eyeball of a patient along the retina.

The outer diameter of the center of the needle tube 1 is 1.5 mm or more and 3.5 mm or less.

The needle tube 1 has transparency that allows a tissue moving inside the needle tube 1 to be visually recognized from the outside. The transparency of the needle tube 1 can be adjusted by appropriately setting the forming material, wall thickness, and the like of the needle tube 1.

The tip 10 (the needle tube 1, the connecting portion 2, and the blade portion 3) according to the present embodiment is formed from at least one type of resin of polyolefin resin (polypropylene, polyethylene, polymethylpentene), cycloolefin resin [cycloolefin polymer (COP), cycloolefin copolymer (COC)], polyester resin (polyethylene terephthalate), fluorine resin [polytetrafluoroethylene (PTFE), perfluoroalkoxyalkane (PFA), perfluoroethylene-propene copolymer (FEP), ethylene tetrafluoroethylene copolymer (ETFE), polyvinylidene fluoride (PVDF), polychlorotrifluoroethylene (PCTFE), or ethylene chlorotrifluoroethylene copolymer (ECTFE)], polycarbonate, and polyimide.

The needle tube 1, the connecting portion 2, and the blade portion 3 may be integrally molded by using a mold with the above-described resin as a material, or may be individually molded and joined with an adhesive or the like.

Figure 3 is a perspective view schematically illustrating an example of the schematic configuration of a tissue to be transplanted by the tip 10 according to the present embodiment.

A tissue T illustrated in Figure 3 is a retina tissue, and has a minor axis D4 of 400 µm or more and 800 µm or less, and a thickness H of 400 µm or more and 600 µm or less. A major axis D5 is about 1200 µm. The tip 10 according to the present embodiment is configured such that the tissue T can move inside the needle tube 1, with the major axis D5 of the tissue T along the longitudinal direction of the needle tube 1, the minor axis D4 of the tissue T along the major axis D1 direction of the front end portion 12 of the needle tube 1, and the thickness H of the tissue T along the minor axis D2 direction of the front end portion 12 of the needle tube 1 (the inner diameter of the needle tube 1 is set to a dimension that does not inhibit the movement of the tissue T). When the tissue T moves in the front end portion 12 of the needle tube 1, the minor axis D4 and thickness H of the tissue T may be reversibly changed (may contract).

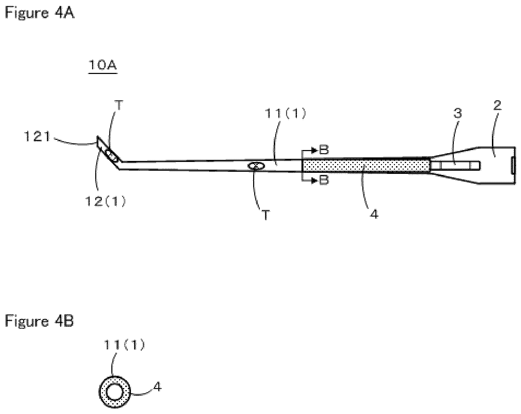

Figures 4A and 4B are diagrams illustrating the schematic configuration of a modification of the tip 10 according to the present embodiment. Figure 4A is a side view illustrating the schematic configuration of a tip 10A according to the modification (a diagram seen from a direction perpendicular to a flat surface that passes the main body portion 11 and the front end portion 12 of the needle tube 1 included in the tip 10A). Figure 4B is an enlarged cross-sectional view taken along the arrows BB illustrated in Figure 4A.

The tip 10A according to the modification illustrated in Figures 4A and 4B is different from the tip 10 only in that a stopper 4 is included in the main body portion 11 of the needle tube 1. The stopper 4 has a function of preventing the movement of the tissue T toward the rear end of the needle tube 1. Specifically, in the example illustrated in Figures 4A and 4B, a blunt needle (for example, a 25 G non-bevel needle) that is inserted into the main body portion 11 of the needle tube 1, and that has an inner diameter through which the tissue T cannot pass (cannot pass even if the tissue T contracts) is used as the stopper 4.

According to the tip 10A according to the modification, since the tissue T does not move toward the rear end beyond the position at which the stopper 4 is provided (does not move to the right of the BB line illustrated in Figure 4A), the tissue T is not excessively sucked inside the needle tube 1, and the tissue T can be more reliably transplanted.

Note that although Figures 4A and 4B exemplify the configuration in which the blunt needle inserted into the needle tube 1 is used as the stopper 4, the stopper 4 is not limited to this. Various forms can be employed as long as a function of preventing the movement of the tissue T toward the rear end of the needle tube 1 can be achieved. For example, it is also possible to provide a reduced diameter portion in the needle tube 1 itself, and to cause this reduced diameter portion to function as the stopper 4.

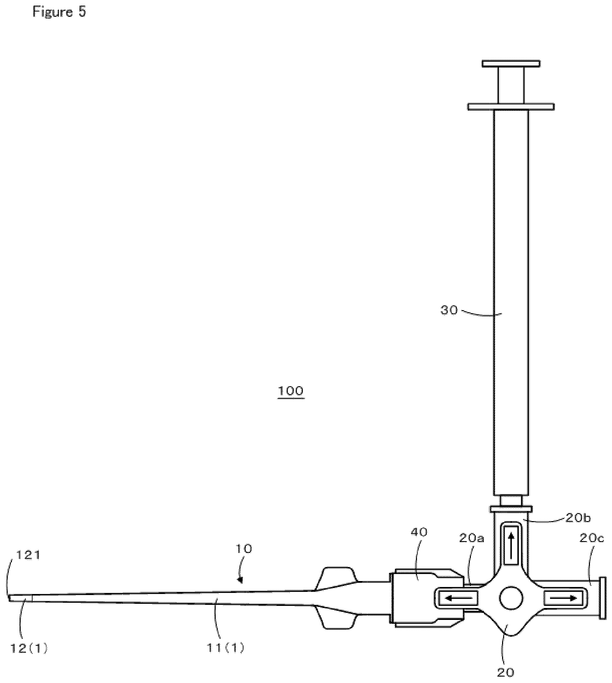

Figure 5 is a plan view illustrating the schematic configuration of a tissue transplantation device according to an embodiment of the present invention.

As illustrated in Figure 5, a tissue transplantation device 100 according to the present embodiment includes the stopcock 20, the tip 10 according to the present embodiment connected to a port of the stopcock 20, and a syringe 30 connected to another port of the stopcock 20 (for example, a 1-ml syringe or a 0.5-ml syringe). Note that, in the present embodiment, at least the tissue T and the tip 10 constitute a retina tissue transplantation kit. In addition to the tissue T and the tip 10, the retina tissue transplantation kit may further include at least one or more of the syringe 30, and an injection kit and a tube, which will be described later.

The tissue transplantation device 100 according to the present embodiment includes, as the stopcock 20, a three-way stopcock including three ports 20a, 20b, and 20c. However, the present invention is not limited to this, and can also adopt a configuration in which a two-way stopcock is included as the stopcock 20. In the example illustrated in Figure 5, the tip 10 is connected to the port 20a via a lock adapter 40, and the syringe 30 is connected to the port 20b.

Hereinafter, an example of a transplantation method of the tissue T (retina tissue) using the tissue transplantation device 100 will be described.

First, general vitreous stalk microscopic dissection is performed by using a cataract/vitreous surgery device (for example, a CONSTELLATION vision system manufactured by Alcon, Inc.). On this occasion, if necessary, posterior vitreous detachment is caused.

Next, an injection kit (for example, a MedOne injection kit) is connected to the cataract/vitreous surgery device, this injection kit is filled with a balanced salt solution, and a cannula (for example, a MedOne cannula, Japanese medical device certification number: 226AFBZI00075000) is connected to the injection kit. Then, using a liquid injection function of the cataract/vitreous surgery device, the balanced salt solution filled in the injection kit is injected into a space under a retina via the cannula, and a localized retinal detachment is created. Then, a portion of the detached retina is partially cut open by vitreous scissors or the like to form a transplant wound.

On the other hand, the tissue T to be transplanted is moved from a receiving container into a petri dish to be floated. Then, in a state where the port 20a connected to the tip 10 of the tissue transplantation device 100 and the port 20b connected to the syringe 30 are turned on (opened) as illustrated in Figure 5, the front end 121 of the tip 10 is brought close to the tissue T floating within the petri dish. Next, the tissue T is sucked inside the needle tube 1 (for example, to the center of the needle tube 1) of the tip 10 by pulling the syringe 30. Note that, before pulling the syringe 30 to suck the tissue T, it is preferable to perform priming by filling the inside of the needle tube 1 with an eye irrigation solution for ophthalmic surgery. After sucking the tissue T inside the needle tube 1, at least the port 20b connected to the syringe 30 is turned off (closed), and the syringe 30 is removed from the port 20b. Note that, in order to effectively suppress the movement of the tissue T due to the suction force generated when removing the syringe 30 from the port 20b, or the discharge pressure caused by the movement of the air, it is preferable to turn off (close) both the port 20b connected to the syringe 30 and the remaining port 20c, and to remove the syringe 30 from the port 20b.

Next, in a state where the tissue T is still sucked inside the needle tube 1 of the tip 10, an injection kit (for example, a MedOne injection kit) is connected to the remaining port 20c of the stopcock 20, the aforementioned cataract/vitreous surgery device is connected to the injection kit, and the port 20a connected to the tip 10 and the port 20c connected to the injection kit are turned on (opened). Note that, when connecting the injection kit to the port 20c, it is preferable to fill a dead space of the port 20c with the eye irrigation solution for ophthalmic surgery. Additionally, it is also possible to connect the injection kit to the port 20c via a tube, rather than directly connecting the injection kit to the port 20c.

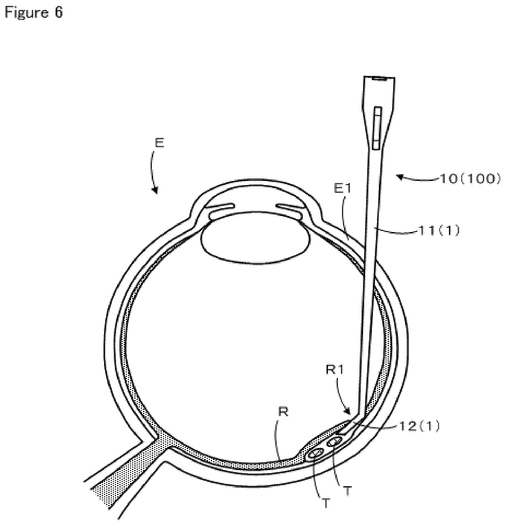

Next, as illustrated in Figure 6, the needle tube 1 of the tip 10 is inserted into an eyeball E of a patient from an incision created in a sclera E1 of the eyeball E, and the front end portion 12 is caused to reach the aforementioned transplant wound R1 created in a retina R. Then, after inserting the front end portion 12 of the needle tube 1 into a space under the retina from the transplant wound R1, the tissue T is discharged to the space under the retina from inside the needle tube 1 by operating the injection kit using the liquid injection function of the cataract/vitreous surgery device. Note that, if necessary, the position of the tissue T after discharging may be adjusted by using subretinal forceps or the like. With the above operation, the tissue T can be transplanted under the retina in the eyeball E.

Hereinafter, a description will be given of an example of test results evaluating ease of transplantation by actually performing, by a doctor, transplant surgeries of the tissue T, with the use of the tip 10 according to the present embodiment, a tip described in Non Patent Literature 1, and a tip prototyped in the process arriving at the present invention, respectively.

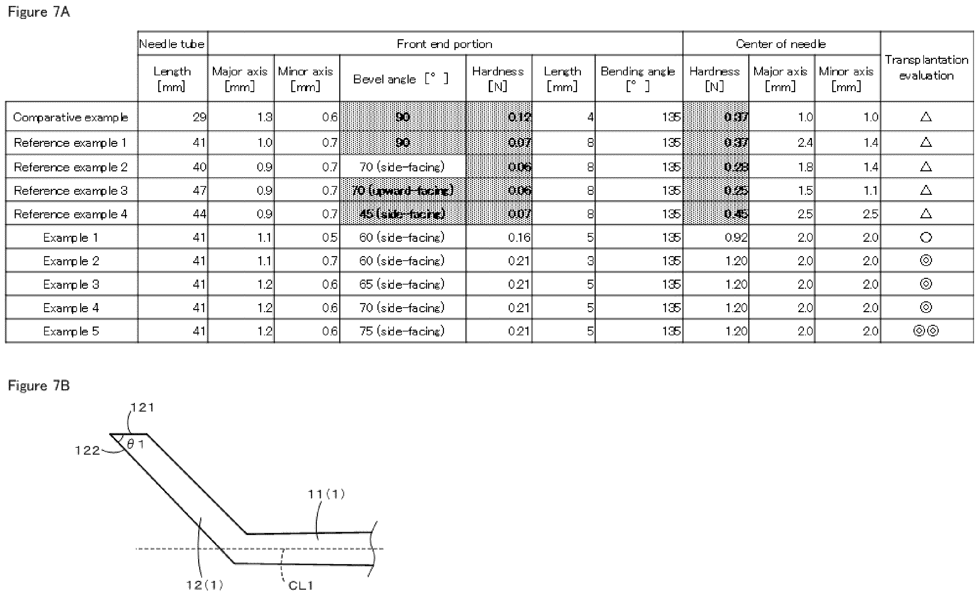

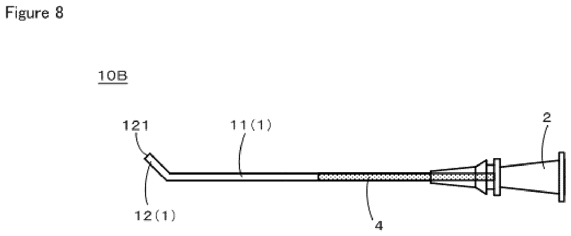

Figures 7A and 7B are diagrams describing the content of evaluation tests. Figure 7A is a diagram illustrating the conditions and evaluation results of each of the tips. Figure 7B is a diagram describing the meaning of a front end of a front end portion of a tip being "an upward-facing bevel surface". Figure 8 is a side view illustrating the schematic configuration of the tip described in Non Patent Literature 1 (a diagram seen from a direction perpendicular to a flat surface that passes a main body portion and a front end portion of a needle tube included in the tip).

"Comparative Example" illustrated in Figure 7A is the conditions and evaluation result of the tip described in Non Patent Literature 1, "Reference Example 1" to "Reference Example 4" are the conditions and evaluation results of the prototyped tip, and "Example 1" to "Example 5" are the conditions and evaluation results of the tip 10 according to the present embodiment.

As illustrated in Figure 8, similar to the tip 10 according to the present embodiment, a tip 10B according to Comparison Example includes the needle tube 1 (the main body portion 11 and the front end portion 12) and the connecting portion 2. Additionally, similar to the tip 10A according to the aforementioned modification, the stopper 4 is provided. However, the tip 10B according to Comparison Example does not include the blade portion 3. In addition, the front end 121 of the front end portion 12 of the tip 10B according to Comparison Example is not a bevel surface, but a surface extending in a direction perpendicular to the longitudinal direction (extending direction) of the front end portion 12. In Figure 7A, although the bevel angle of Comparative Example is written as 90° for convenience of illustration, this means that the front end 121 extends in the direction perpendicular to the longitudinal direction of the tip portion 12 as described above. The same applies to Reference Example 1 illustrated in Figure 7A.

Additionally, although "70 (upward-facing)" is written in the column of "Bevel angle [°]" of Reference Example 3 illustrated in Figure 7A, "upward-facing" means that the front end 121 of the front end portion 12 is an upward-facing bevel surface as illustrated in Figure 7B. The upward-facing bevel surface means a surface extending in a direction substantially parallel to the main body portion 11 of the needle tube 1 (a direction substantially parallel to the central axis CL1 of the main body portion 11 of the needle tube 1). Then, "70 (upward-facing)" written in the column of "Bevel angle [°]" means that the acute angle Θ1 formed by the front end 121 (upward-facing bevel surface) and the external surface 122 of the front end portion 12 of the needle tube 1 that is adjacent to the front end 121 is 70°.

Note that "(side-facing)" written in the columns of "Bevel angle [°]" of Reference Example 2, Reference Example 4, and Examples 1 to 5 illustrated in Figure 7A means that, as mentioned above with reference to Figure 1C, the front end 121 of the front end portion 12 is a bevel surface extending in a direction substantially perpendicular to the main body portion 11.

In Figure 7A, the numerical values in the hatched columns mean that the conditions required for the tissue transplantation resin tip according to the present invention are not satisfied.

Additionally, "Δ" written in the column of "Transplantation evaluation" illustrated in Figure 7A means that the transplantation of the tissue T under the retina was somewhat difficult. "O" means that the transplantation of the tissue T under the retina was generally successful. " 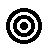 " means that transplantation of the tissue T under the retina was successful without problems. " 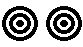 " means that the transplantation of the tissue T under the retina was the most successful without problems.

Specifically, in Comparative Example, Reference Example 1 to Reference Example 4, Example 1, and Example 2, the transplantation of the tissue T under a retina of a monkey was evaluated by using the tissue transplantation resin tip. In Examples 3 to 5, the transplantation of the tissue T under a retina of an excised pig eye was evaluated by using the tissue transplantation resin tip.

As illustrated in Figure 7A, when the tips 10 according to the present embodiment was used (Examples 1 to 5), the transplantation of the tissue T under the retina was generally successful, or successful without problems. Especially, it was found out that, when the tissue transplantation resin tips of Examples 2 to 5 were used, the transplantation of the tissue T under the retina to a large animal was particularly successful.

That is, it was confirmed that, according to the tissue transplantation resin tip according to the present invention, the tissue T sucked inside could be easily transplanted.

### [Reference Signs List]

1...needle tube
2... connecting portion
3...blade portion
10, 10A ...tissue transplantation resin tip
11...main body portion
12...front end portion
100...tissue transplantation device
111...rear end of needle tube
121...front end of needle tube
L1...length of needle tube
D1...major axis of cross-sectional external shape of front end portion of needle tube
D2...minor axis of cross-sectional external shape of front end portion of needle tube
T...tissue
θ1...bevel angle
θ2...bending angle

## Claims

1. A tissue transplantation resin tip to be used for transplanting a tissue under a retina in an eyeball, the tissue transplantation resin tip comprising:
a needle tube to be inserted into an eyeball,
wherein the needle tube includes a main body portion that extends linearly, and a front end portion that is bent with respect to the main body portion and extends linearly, and that discharges the tissue from a front end,
a length of the needle tube is 25 mm or more and 50 mm or less,
a cross-sectional external shape of the front end portion is an oval shape having a major axis of 0.8 mm or more and 1.5 mm or less, and a minor axis of 0.5 mm or more and 1.0 mm or less,
the front end of the front end portion is a bevel surface that extends in a direction substantially perpendicular to the main body portion, and that has a bevel angle of 50° or more and 85° or less, and
a hardness of the front end portion is 0.15 N or more and 0.30 N or less, and a hardness of a center of the needle tube is 0.80 N or more and 1.50 N or less.

2. The tissue transplantation resin tip according to claim 1,
wherein the hardness of the front end portion is 0.20 N or more and 0.30 N or less.

3. The tissue transplantation resin tip according to claim 1 or 2,
wherein a length of the front end portion is 2 mm or more and less than 6 mm.

4. The tissue transplantation resin tip according to any one of claims 1 to 3,
wherein the front end portion is bent so as to form an angle of 120° or more and 160° or less with respect to the main body portion.

5. The tissue transplantation resin tip according to any one of claims 1 to 4,
wherein an outer diameter of the center of the needle tube is 1.5 mm or more and 3.5 mm or less.

6. The tissue transplantation resin tip according to any one of claims 1 to 5,
wherein the needle tube has transparency that allows the tissue moving inside the needle tube to be visually recognized from an outside.

7. The tissue transplantation resin tip according to any one of claims 1 to 6,
wherein the tissue transplantation resin tip is formed from at least one type of resin of polyolefin resin, cycloolefin resin, polyester resin, fluorine resin, polycarbonate, and polyimide.

8. The tissue transplantation resin tip according to any one of claims 1 to 7, comprising:
a stopper that prevents movement of the tissue toward a rear end of the needle tube in the main body portion.

9. The tissue transplantation resin tip according to any one of claims 1 to 8,
wherein the tissue is a retina tissue, and
the retina tissue having a minor axis of 400 µm or more and 800 µm or less, and a thickness of 400 µm or more and 600 µm or less can move inside the needle tube.

10. A tissue transplantation device, comprising:
a stopcock;
the tissue transplantation resin tip according to any one of claims 1 to 9 configured to be connected to a port of the stopcock; and
a syringe configured to be connected to another port of the stopcock.

11. A retina tissue transplantation method of transplanting a retina tissue under a retina in an eyeball of a patient who suffers from a disease based on a disorder of or a damage to the retina tissue, the retina tissue transplantation method comprising:
a step of sucking the retina tissue inside the needle tube of the tissue transplantation resin tip by pulling the syringe using the tissue transplantation device according to claim 10;
a step of inserting the needle tube that has sucked the retina tissue into a space under the retina in the eyeball of the patient; and
a step of discharging the retina tissue to the space under the retina from inside the needle tube.

12. A retina tissue transplantation kit, comprising:
a retina tissue; and
the tissue transplantation resin tip according to any one of claims 1 to 8.
